# EUROPEAN PATENT APPLICATION

(11) **EP 2 518 512 A1**
(43) Date of publication of application: **31.10.2012**
(21) Application number: 11164223.7
(22) Date of filing: 29.04.2011
(51) Int. Cl.: G01N 33/92, G01N 33/68

(54) **Method for indicating a renal disease in a patient by determining at least one protein in isolated HDL**

(71) Applicant: Medizinische Universität Wien, 1090 Wien (AT)
(72) Inventor: Weichhart, Thomas, 3071 Böheimkirchen (AT); Säemann, Marcus, 1150 Wien (AT)
(74) Representative: Sonn & Partner Patentanwälte

(57) **Abstract**

The invention discloses a method for indicating a renal disease in a patient comprising the following steps:
- providing a blood, serum or plasma sample from said patient,
- isolating high density lipoprotein (HDL) from said sample to obtain isolated HDL,
- determining the protein content of at least one protein of the following group of proteins in said isolated HDL: Surfactant protein B (SP-B), ApoC-II, Serum amyloid A, AMBP, ApoH, Serpin A12, Serotransferrin, Pigment epithelium-derived factor (PEDF), ADAM30 and Complement factor D, preferably at least one of SP-B, ApoC-II, Serum amyloid A and AMBP, especially SP-B, wherein a renal disease is indicated in said patient if the determined protein content of said at least one protein is elevated in said isolated HDL compared with a healthy subject.

## Description

End-stage renal disease (ESRD) represents a major health problem and requires maintenance dialysis. Mortality remains above 20 percent per year in the United States with the use of dialysis, with more than half of the deaths related to cardiovascular disease (CVD). Atherosclerosis as underlying cause for cardiovascular morbidity and mortality is increased up to 30 fold in patients with ESRD, as well as in milder degrees of renal dysfunction. Atherosclerosis in ESRD is thought to be driven by several factors including inflammation, oxidative stress and dyslipidemia. Dyslipidemia in ESRD patients is characterized by a dysregulation of the synthesis and activity of high-density lipoprotein (HDL) leading to decreased plasma levels of HDL cholesterol (HDL-C). Many epidemiological studies have documented an inverse relationship between HDL levels and the progression of atherosclerosis and hence CVD in the general population. The atheroprotective and anti-inflammatory properties of high-density lipoprotein (HDL) are therefore lost in patients with ESRD; however, the underlying reasons are unclear. A wide range of possible mechanisms for the atheroprotective function of HDL has been proposed such as reverse cholesterol transport (RCT), the reduction of oxidative stress, or potent anti-inflammatory effects including its ability to inhibit the expression of adhesion molecules on endothelial cells. On the other hand, HDL might lose its anti-atherogenic properties by chemical modifications such as oxidation, which negatively affects RCT and other events associated with the development of atherosclerosis. Hence, oxidized HDL can be detected in lesions and plasma of individuals at increased atherosclerotic risk. Various investigators have proposed that oxidative stress might constitute a strong risk factor of increased cardiovascular morbidity and mortality of ESRD patients, and it was suggested that oxidized HDL exists in maintenance hemodialysis patients and is associated with malnutrition and inflammation.

Despite reduced serum HDL-C concentrations in ESRD patients a clear association of HDL concentration with survival could not be demonstrated. However, the anti-inflammatory function of HDL as assessed by the ability of HDL to inhibit low density lipoprotein (LDL) oxidation is defective in ESRD patients and correlates with overall survival. Indeed, the conversion of anti-inflammatory to proinflammatory HDL has also been proposed to represent a novel risk factor for the progression of CKD to ESRD. In this line, the levels of apolipoprotein A1 (apoA-I) in HDL are reduced, whereas serum amyloid A (SAA) and secretory phospholipase A2 (sPLA2) are enhanced in ESRD-HDL (Saemann et al., Eur J Clin Invest 40 (2010): 1134-1143).

Recently, mass spectrometry (MS) has been utilized to elucidate the proteome of HDL. These studies revealed that the protein cargo of HDL seems to be a major determinant of the anti-atherogenic and anti-inflammatory function of HDL. For example, approximately 50% of the proteins associated with HDL are implicated in the acute-phase, the complement as well as the innate immune response (Vaisar et al., J Clin Invest 117 (2007): 746-756; US 2007/099242 A1).

Screening and diagnosing methods for renal diseases are still problematic and there is a high need for more efficient screening strategies and better screening tests (Hallan et al., J Nephr 23 (2010): 147-155). There has been increasing interest in the role of biomarkers that can identify early stages of disease or injury that correlate well with progression and potentially response to therapy. The underlying premise is that early detection and understanding of disease can then lead to interventions that stop or retard disease/injury. Applying new technologies of genomic analysis (e.g., RNA subtraction or DNA mi-croarrays) and proteomic approaches to identify novel proteins that encode for renal injury are ongoing (Chaudhary et al., Curr Diab Rep 10 (2010): 37-42; US 2003/003588 A1; EP 1 255 114 A1; WO 2010/048346 A1).

It was therefore an object of the present invention to provide means for (assisting in) diagnosing renal diseases and evaluating the status of patients with renal diseases or patients which are suspected to have or suspected of being at risk for having renal diseases.

Therefore, the present invention provides a method for indicating a renal disease in a patient comprising the following steps:
- providing a blood, serum, plasma sample from said patient,
- isolating high density lipoprotein (HDL) from said sample to obtain isolated HDL,
- determining the protein content of at least one protein of the following group of proteins in said isolated HDL: Surfactant protein B (SP-B), ApoC-II, Serum amyloid A, AMBP, ApoH, Serpin A12, Serotransferrin, Pigment epithelium-derived factor (PEDF), ADAM30 and Complement factor D, preferably at least one of SP-B, ApoC-II, Serum amyloid A and AMBP, especially SP-B, wherein a renal disease is indicated in said patient if the determined protein content of said at least one protein is elevated in said isolated HDL compared with a healthy subject.

With the present invention it could be shown that the "HDL status" of a patient, i.e. the quality regarding the protein and lipid composition rather than the mere quantity of HDL is critical for the anti-atherosclerotic and anti-inflammatory potency in ESRD. Although oxidative alterations of HDL are directly linked with increased cardiovascular complications, HDL of ESRD patients requiring maintenance hemodialysis is not significantly altered with respect to oxidative status. Surprisingly, it was found that the HDL of ESRD is not oxidized or more vulnerable to oxidation compared to HDL from healthy controls. However, a severely distorted protein composition was identified in ESRD patients, on which the diagnostic method according to the present invention is based. This also includes novel biomarkers for use in future clinical studies for patients with renal disease or patients which are suspected to have or suspected of being at risk for having (or acquiring) renal diseases.

In the course of the present invention, a comprehensive analysis of all proteins present in HDL of patients with renal diseases, especially ESRD patients, has been conducted as well as a precise investigation of the oxidation status of such patients' HDL. Using a mass spectrometry-based approach in total 64 HDL-associated proteins were identified, several of which were previously not known to reside in HDL. The present data show that HDL is a major regulator of heme/iron metabolism. Notably, some proteins were selectively present or significantly enriched in HDL of ESRD patients such as surfactant-protein B (SP-B), apolipoprotein C-II, serum amyloid A, serotransferrin, or α-1-microglobulin/bikunin precursor. These findings were further validated in an independent ESRD cohort showing that these proteins are suitable novel markers for the detection of renal diseases, especially ESRD. Strikingly, some of these proteins including SP-B and serotransferrin were already enriched in earlier stages of the disease, especially in the HDL of stage 4 CKD. Surprisingly, it was not possible to detect any oxidized HDL in plasma from ESRD patients showing that HDL is not a principal target for the putative highly elevated oxidative stress during uremia.

The present invention provides a suitable indication which allows or assists a person with the appropriate medical skills to make a diligent diagnosis for an individual patient. It is evident that the final diagnosis has to include the personal specificities and etiology of each patient; however, the indicativeness of the qualitative composition of HDL according to the present invention, especially with respect to the renal disease markers disclosed, is shown in the example section of the present invention.

Accordingly, a renal disease is indicated (i.e. to be diagnosed) according to the present invention if one or more proteins selected from SP-B, ApoC-II, Serum amyloid A, AMBP, ApoH, Serpin A12, Serotransferrin, PEDF, ADAM30 and Complement factor D, preferably from of SP-B, ApoC-II, Serum amyloid A and AMBP, especially SP-B, is present in a significantly increased amount in HDL in the blood of a patient, compared to the amount of this protein in HDL in the blood of a healthy person, i.e. a person who does not have such renal disease (and who does not have another disorder which influences the protein composition in HDL).

It is essential for the present invention to assess the marker function of the protein composition in HDL, because surprisingly, the markers according to the present invention do not exhibit the marker function in whole blood, serum or plasma (or in other body fluids analysed so far. This means that analysis of the blood derived sample will not lead to the results according to the present invention. It is absolutely necessary to analyse the HDL portion of blood derived patient samples. It is therefore necessary to analyse HDL protein composition in a form which is separated from other blood components and fractions. This can be done by a physical separation of HDL from other components of the blood derived sample preceding the determination step or by methods which allow in situ isolation of HDL whereupon protein composition in HDL may be determined.

According to a preferred embodiment of the present invention isolation of HDL is performed by precipitation, ultracentrifugation, fast protein liquid chromatography (FPLC) or immunoseparation.

Isolation of HDL by precipitation can be carried out by various established precipitation agents and combination of agents e.g. heparin/MnCl₂, dextran sulphate, sodium phosphotungstate-MgCl₂ or PEG. Exemplary methods for performing such precipitations in detail may be as follows:
Heparin-MnCl₂: A stock solution is prepared by diluting a sodium heparin solution with isotonic saline (NaCl) (Wiebe et al., Clin Chem 31 (1985): 746-750). The HDL isolation reagent is prepared by mixing the heparin stock solution with a MnCl₂ solution, where the final concentration of MnCl₂ might be 46 mmol/L or 92 mmol/L. An aliquot of this mixture is dispensed into a serum sample. The resulting mixtures are processed as previously described (Lipid ResearchClinics Program. Manual of laboratory operations. Lipid and lipoprotein analysis, 2nd ed., A Hainline Jr, J Karon, K Lippel, Eds. National Heart, Lung, and Blood Institute, Bethesda, MD 20205, NIH pubi. no 75-628, revised 1982; Kostner, Clin Chem 22 (1976), 695).

Dextran sulfate: A dextran sulfate stock solution is prepared by dissolving dextran sulfate in de-ionized water. The HDL isolation reagent is prepared by mixing equal volumes of the stock dextran sulfate solution and MgCl₂ solution. An aliquot of the working reagent is dispensed into the serum sample. The resulting mixtures were processed as previously described (Kostner, Clin Chem 22 (1976), 695).

Sodium phosphotungstate-MgCl₂ (NAP): For HDL isolation a stock solution of sodium phosphotungstate is prepared by dissolving phosphotungstic acid in a NaOH solution, then adding de-ionized water. The final isolation reagent is prepared by mixing the stock solution with a MgCl₂ solution. An aliquot of the reagent is dispensed into the serum sample.

Polyethylene glycol 6000 (PEG): The PEG-HDL isolation reagent is prepared by dissolving polyethylene glycol 6000 in glycerin buffer to give a final volume of 100 mL. VLDL and LDL are precipitated by mixing the PEG reagent with serum and incubating the resulting mixture at ambient temperature before centrifugation. In another possibility, the HDL fraction used in the cholesterol efflux capacity assay may be obtained by depleting serum of apolipoprotein B particles (chylomicrons, VLDL, and LDL) using polyethylene glycol (PEG) precipitation Treatment of fresh sera with polyethylene glycol 6000 at a final concentration of 100 g/l produces selective precipitation of apoliprotein B-containing particles leaving HDL in the supernatant. This method yielded recovery of more than 97% of apolipoprotein A-I-containing HDL particles and less than 2% of apolipoprotein B containing low-density lipoprotein (LDL) and very-low-density lipoprotein (VLDL) particles.

Obtaining HDL by ultracentrifugation has been the "classical" way of obtaining HDL; in fact, the definition of HDL is based upon the behavior in the ultracentrifuge (as having a density of 1.063 to 1.210 g/mL and a size of 5 to 17 nm; this further shows that the composition of HDL is not uniform). Accordingly, HDL isolation by ultracentrifugation could be performed as follows: HDL is isolated from fresh human plasma by sequential ultracentrifugation at a density of 1.06 - 1.21g/mL, preferably 1.12 - 1.21 (Havel et al., J Clin Invest 34 (1955): 1345-53). The density of plasma was raised by addition of KBr (Merck, Darmstadt, Germany) to 1.125 g/mL, and ultracentrifugation was carried out for 12 h at 20°C in a 50.4 Ti rotor at 50,000 rpm using an Optima L-90-K ultracentrifuge (Beckman, Fullerton, CA). After removing the supernatant with the larger lipoproteins, the density of the infranate fraction was raised to 1.210 g/mL by further addition of KBr. HDL were then collected from the top of each polycarbonate thick wall centrifuge tube (Beckman) after another centrifugation step in the 50.4 Ti rotor at 50,000 rpm for 12 h.

An example for FPLC isolation of HDL is the following procedure: Total HDL separations by FPLC are performed on two Superose 6 HR 10/30 columns in series (Amersham Pharmacia Biotech Inc., Orsay, France). Before application, the columns are injected with 25mL of a 20% ethanol solution, afterwards cleaned with 25mL de-ionized water and then equilibrated with 50mL PBS at a flow rate of 0.5 mL/min. Two hundred microliters of plasma are injected and elution is performed at a flow of 0.35 mL/min. Approximately 0.35 mL fraction are collected. An entire profile is completed within 60-100 min.

Immunoseparation can e.g. be performed in the following way: HDL may be isolated from serum by immunoprecipitating HDL via antibodies directed against HDL (e.g. apoAI or the entire HDL molecule) and protein A/G Sepharose beads. Briefly, the serum sample is first reacted with pre-titered primary antibody (e.g. apoAI or the entire HDL molecule (chicken anti-human HDL)) overnight at 4°C with gentle agitation on a rotator. After the incubation, the precipitating reagent (recombinant protein G or protein A attached to agarose beads) is added in amounts sufficient to precipitate the entire primary antibody based on the manufacturer's recommendation. The mixture is further incubated for 1 h at room temperature with rotation. The supernatant and precipitate are separated by centrifugation and the precipitate is washed thoroughly with PBS.

However, as already stated, the methods for isolating HDL are well available to a person skilled in the art.

The same holds true for the determination of the protein content of each of the specific proteins in the HDL fraction according to the present invention. All proteins referred to as specific HDL renal disease marker in the present invention are known in principle; the usual and appropriate methods for their determination can therefore be immediately applied by a person skilled in the art. Accordingly, this determination of the at least one protein of said group of proteins for the diagnose information according to the present invention is preferably performed by an enzyme linked immunoassay (ELISA), electrophoresis, immunoblotting, mass spectroscopy, immunoprecipitation, Multiplex bead-based assays (such as e.g. Luminex technology), or combinations of said methods. These are standard assays which can easily applied and optimised for each of the HDL proteins by using ordinary skills.

The variation of the HDL protein composition according to the present invention can be helpful in diagnosing a variety of renal diseases and predict future clinical events, such as cardiovascular events, pulmonary damage etc. It is even possible to track early stages of renal diseases with the methods according to the present invention. Preferably, the renal disease to be diagnosed with the present invention is chronic kidney disease stage 3, chronic kidney disease stage 4 or end-stage renal disease (ESRD; chronic kidney disease stage 5). Additionally, future clinical events such as cardiovascular diseases or pulmonary damage etc. may be predicted.

The presence of an observable difference of the HDL profile is indicative for a renal disease according to the present invention. A specific difference is an elevated protein content of a protein selected from the group of the specific markers according to the present invention (SP-B, ApoC-II, Serum amyloid A, AMBP, ApoH, Serpin A12, Serotransferrin, PEDF, ADAM30 and Complement factor D, preferably from of SP-B, ApoC-II, Serum amyloid A and AMBP, especially SP-B). An "elevated" protein content (indicating a renal disease) is a content which significantly differs from the content in HDL of a healthy subject. A "healthy subject" according to the present invention can be a person or a group of persons which have no renal disease (as verified by other accepted diagnostical methods). The difference in protein content should be significant, preferably statistically significant according to established medical standards, especially by e.g. Student's t test (Student's unpaired 2-tailed t test). However, statistical significance is only one possibility to finally determine the "elevated" status (yet a preferred one), however, especially for low case numbers, statistical significance is not mandatory; a clear observable trend can also be sufficient.

The quantitative amount which is needed to establish an "elevated" status of a given protein depends on various factors, such as presence and amount in healthy HDL, variability in individual HDL or variability in limited groups of individuals, variability of the detection method (e.g. sensitivity and specificity), etc..

In a standardised and practically applied assay according to the present invention, this assay should fulfil all necessary requirements of diagnostic kits or methods, if larger numbers of patients have to be analysed.

A preferred definition for an elevated level of a specific HDL marker protein can be given by the "peptide index" as defined in the example section. With this definition, the determined protein content of said at least one protein is regarded as being elevated in said isolated HDL compared with a healthy subject by a peptide index of at least 0.2, preferably at least 0.4, especially at least 0.6.

Another preferred definition of the elevated status of the protein content of a specific marker protein in HDL can be defined by the relative difference compared to the "healthy" status. Accordingly, the protein content in HDL is preferably regarded as being elevated, if the determined protein content of said at least one protein in the isolated HDL is at least 30%, especially by at least 50%, especially by at least 100%, higher than in HDL of a healthy subject.

According to another aspect, the present invention concerns a kit comprising
- a container for a blood, serum or plasma sample,
- means for isolation of HDL from a blood, serum or plasma sample,
- one or more detection reagent or determination means for determining the protein content of at least one protein of the following group of proteins in isolated HDL of a sample: Surfactant protein B (SP-B), ApoC-II, Serum amyloid A, AMBP, ApoH, Serpin A12, Serotransferrin, Pigment epithelium-derived factor (PEDF), ADAM30 and Complement factor D, preferably at least one of SP-B, ApoC-II, Serum amyloid A and AMBP, especially SP-B.

The kit according to the present invention is a preferred tool for carrying out the methods according to the present invention. The kit can be present in a standardised form as a package and, preferably ready for use. The kit according to the present invention preferably contains standardisation means for determining said protein content, preferably sample(s) of the isolated proteins, a healthy blood, plasma or serum sample and/or a renal disease blood, plasma or serum sample, or written information indicating the protein contents in a healthy and/or a renal disease sample. These standard samples may be individual samples from an individual or a group (or population) of individuals with an ascertained "renal disease" or "healthy" status. These standardisation samples can be used for performing the comparison with the test samples to be investigated by the methods according to the present invention. Alternatively (or in addition) the test kit may also comprise standard amount of proteins in HDL indicating a "renal disease" and/or "healthy" status ("healthy, of course, is - according to the present invention - always limited to a "no renal disease" status; presence of other diseases (e.g. a tumour disease or a cardiovascular disease) in the same patient may disturb the outcome and relevance of the diagnostic indication of the present invention; this is, however, known to a person skilled in the art and a physician will always take the presence of other diseases or disorders in the individual patient into account when the final diagnosis is made.

The kit according to the present invention preferably contains a suitable substrate for determining the protein content of at least one protein of the marker proteins in isolated HDL of a sample. Preferably, such a substrate is provided in association with a detection reagent, preferably a membrane, a filter, a chip, a bead, a gel, a well, a tube, a plate, a pin, a channel, a pore, a trench, a microparticle or a capillary.

According to another aspect, the present invention relates to a method for evaluating the renal status in a patient having or being at risk of having a renal disease comprising the following steps:
- providing a blood, serum or plasma sample from said patient,
- isolating high density lipoprotein (HDL) from said sample to obtain isolated HDL,
- determining the protein content of at least one protein of the following group of proteins in said isolated HDL: Surfactant protein B (SP-B), ApoC-II, Serum amyloid A, AMBP, ApoH, Serpin A12, Serotransferrin, Pigment epithelium-derived factor (PEDF), ADAM30 and Complement factor D, preferably at least one of SP-B, ApoC-II, Serum amyloid A and AMBP, especially SP-B, wherein the renal status is evaluated, preferably as being a disease status or a non-diseased status, by comparison of the protein content of said at least one protein determined with the protein content of said at least one protein in a subject with a known renal status.

According to another aspect, the present invention also relates to the use of Surfactant protein B (SP-B), ApoC-II, Serum amyloid A, AMBP, ApoH, Serpin A12, Serotransferrin, Pigment epithelium-derived factor (PEDF), ADAM30 or Complement factor D, preferably at least one of SP-B, ApoC-II, Serum amyloid A or AMBP, especially SP-B, in the diagnosis of a renal disease in a blood, serum or plasma sample.

Another aspect of the present invention is a method for diagnosing a renal disease in a patient comprising the following steps:
- providing a blood, serum or plasma sample from said patient,
- isolating high density lipoprotein (HDL) from said sample,
- analysing the protein composition in said isolated HDL to obtain a HDL protein profile of said sample,
- comparing the HDL protein profile of said sample with a HDL protein profile of a healthy person and with a HDL protein profile of a patient with confirmed renal disease, and
- diagnosing a renal disease if the comparison of the HDL protein profile of said sample corresponds to said HDL protein profile of a patient with confirmed renal disease. Establishment
and comparison of HDL protein profiles of patient samples and HDL protein profiles of healthy persons can easily be performed by various computer programms. Efficiency of such profile comparison increases with the numbers of samples. Preferably, HDL protein profiling is carried out with a protein set comprising at least Surfactant protein B (SP-B), ApoC-II, Serum amyloid A, AMBP, ApoH, Serpin A12, Serotransferrin, Pigment epithelium-derived factor (PEDF), ADAM30 or Complement factor D, preferably at least one of SP-B, ApoC-II, Serum amyloid A or AMBP; however, including the "fingerprint" of other marker proteins may be beneficial for a number of patients.

Preferably, obtaining the HDL protein profile in this method includes the determination of the protein content of at least one protein of the group consisting of Surfactant protein B (SP-B), ApoC-II, Serum amyloid A, AMBP, ApoH, Serpin A12, Serotransferrin, Pigment epithelium-derived factor (PEDF), ADAM30 and Complement factor D, preferably at least one of SP-B, ApoC-II, Serum amyloid A and AMBP, especially SP-B.

According to another aspect, the present invention provides a method for diagnosing a renal disease in a patient comprising the following steps:
- providing a blood, serum or plasma sample from said patient,
- isolating high density lipoprotein (HDL) from said sample,
- determining the presence or absence of SP-B in said isolated HDL,
- optionally analysing the protein content of SP-B in said isolated HDL to obtain a HDL protein profile of said sample,
- diagnosing a renal disease if the presence of SP-B in said isolated HDL is determined.

SP-B has turned out to be present in HDL only in patients with renal disease. Surprisingly, overall content of SP-B in blood is not a marker for renal disease. However, in healthy individuals, SP-B cannot be detected in HDL, it is only present in HDL of patients with renal disease:
Up to about 0.5 - 0.6% of all HDL proteins in ESRD patients is SP-B whereas no signal can be obtained for SP-B in healthy HDL showing that a measureable amount of 0.005 to 0.01% SP-B (of total HDL protein) can already be indicative of (early stage) renal disease in a patient.

Preferably, the following minimal values of the markers according to the present invention (in % of total HDL protein) are
- independently of each other - indicative for a renal disease according to the present invention: SP-B: more than 0.005%, preferably more than 0.01%, especially more than 0.05%; Apo C-II: more than 1.00%, preferably more than 1.20%, especially more than 1.50%; SAA: more than 2.00%, preferably more than 2.30%, especially more than 2.50%; AMBP: more than 0.20%, preferably more than 0.50%, especially more than 1.00%; Apo-H: more than 0.30%, preferably more than 0.50%, especially more than 0.60%; Serpin A12: more than 0.005%, preferably more than 0.05%, especially more than 0.10%; Transferrin: more than 1.50%, preferably more than 1.60%, especially more than 1.70%; PEDF: more than 0.20%, preferably more than 0.25%, especially more than 0.30%; ADAM30: more than 0.005%, preferably more than 0.05%, especially more than 0.10%; CFD: more than 0.10%, preferably more than 0.20%, especially more than 0.30%.

The invention is further disclosed in the following examples and the drawing figures, yet without being limited thereto.
Fig.1 shows cholesterol and lipoprotein profiles of plasma and isolated HDL. Representative FPLC (black lines) and cholesterol profiles (dashed lines) of plasma samples from a (A) healthy and an (B) ESRD subject. Total cholesterol concentrations were analyzed photometrically. (C) Cholesterol elution profiles of HDL isolated by sequential ultracentrifugation.
Fig.2 shows the amount of oxidized apoA-I and apoA-II in HDL and its susceptibility to oxidation of ESRD patients and control subjects. HDL was tested from 15 ESRD patients and 15 controls.
   The amounts of (A) total apoA-I and apoA-II (in nmol/mg HDL protein), (B) oxidized apoA-I and oxidized apoA-II (in nmol/mg HDL protein), (C) total non-oxidized apoA-I/II and oxidized apoA-I/II (in nmol/mg HDL protein), and (D) the molar ratio of oxidized apoA-I/total apoA-I and oxidized apoA-II/total apoA-II were determined by HPLC analysis from freshly isolated HDL (Fresh), HDL that was stored for 3 days at 4° C (3d), and HDL that was stored for 3 days at 4° C and then chemically oxidized by 2,2'-azobis-2-methyl-propanimidamide (3d+AAPH). *P<0.05.

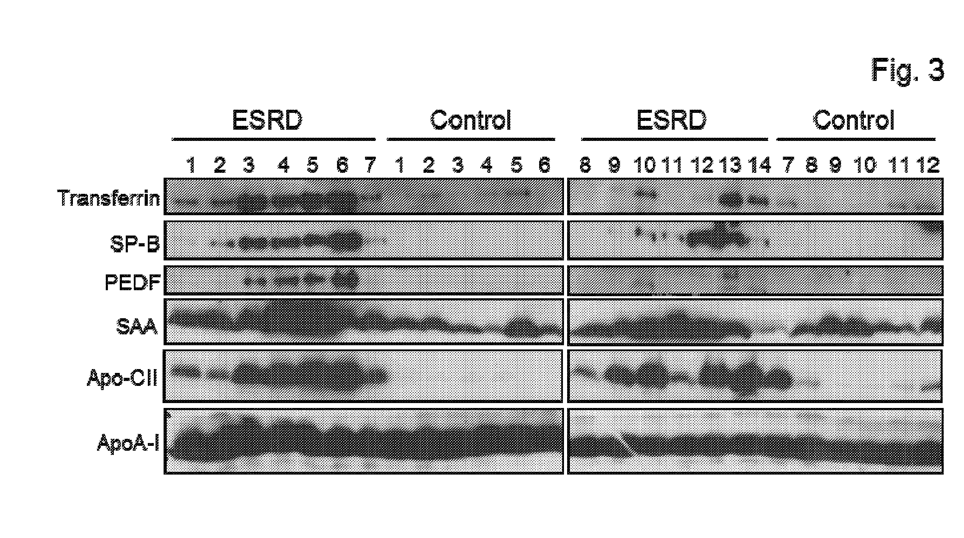

Fig.3 shows the immunodetection of HDL-associated proteins in both ESRD patients and healthy subjects. Shown are immunoblots of the indicated MS-identified HDL proteins in 10 µg of HDL from an independent cohort consisting of 14 ESRD and 12 healthy controls (see Table 1).

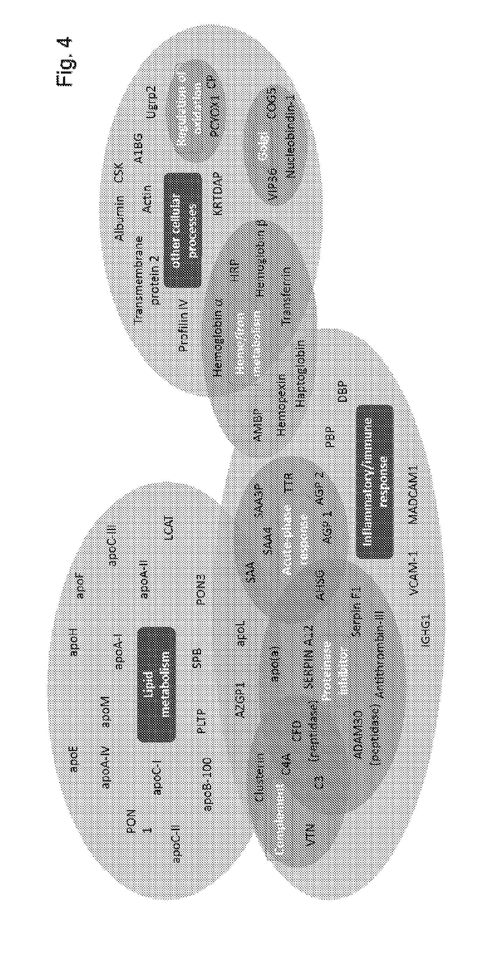

Fig.4 shows gene ontology functional associations of HDL-proteins. Total identified HDL-proteins from 10 healthy controls and 10 ESRD patients were associated with biological functions using Gene Ontology process annotations. HRP, Haptoglobin-related protein; TTR, Transthyretin; IGHG1, Ig gamma-1 chain C region; PLTP, Phospholipid transfer protein; A1BG, alpha-1B-glycoprotein; DBP, Vitamin D-binding protein; AHSG, alpha-2-HS-glycoprotein; VTN, Vitronectin; AGP 2, alpha-1-acid glycoprotein 2; PEDF, pigment epithelium-derived factor; Ugrp2, secretoglobin family 3A member 1; PCYOX1, prenylcysteine oxidase 1; CP, Ceruloplasmin; PBP, platelet basic protein; AGP 1, alpha-1-acid glycoprotein 1; A2GP1, zinc-alpha-2-glycoprotein; SP-B, surfactant protein B; CFD, complement factor D; COG5, COG complex subunit 5; V-CAM1, vascular cell adhesion protein 1.

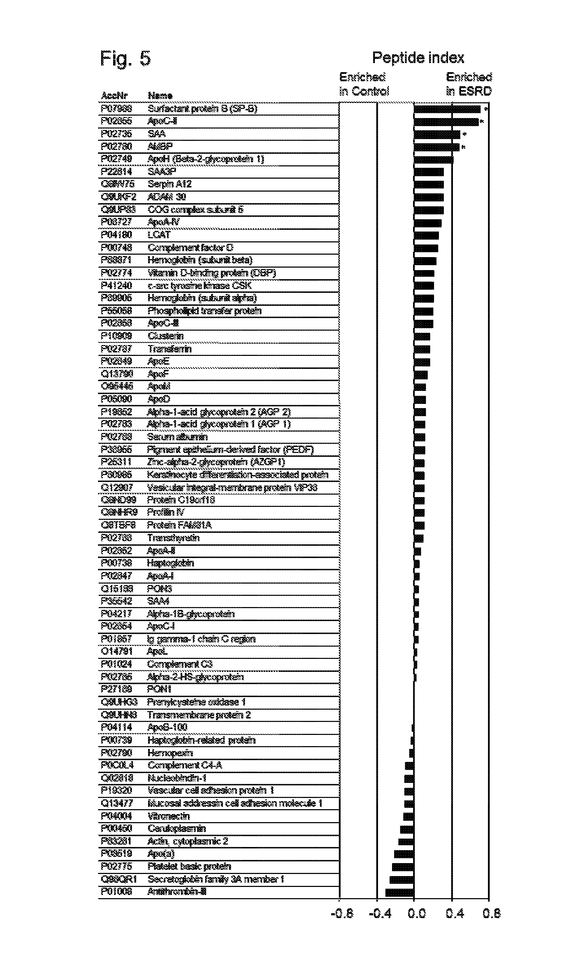

Fig.5 shows the relative abundance of proteins identified by MS from HDL of ESRD patients and healthy controls. Data are from 10 subjects with ESRD and 10 controls. The relative abundance of the HDL-associated proteins was assessed by the peptide index as described in Methods. *P <0.05.

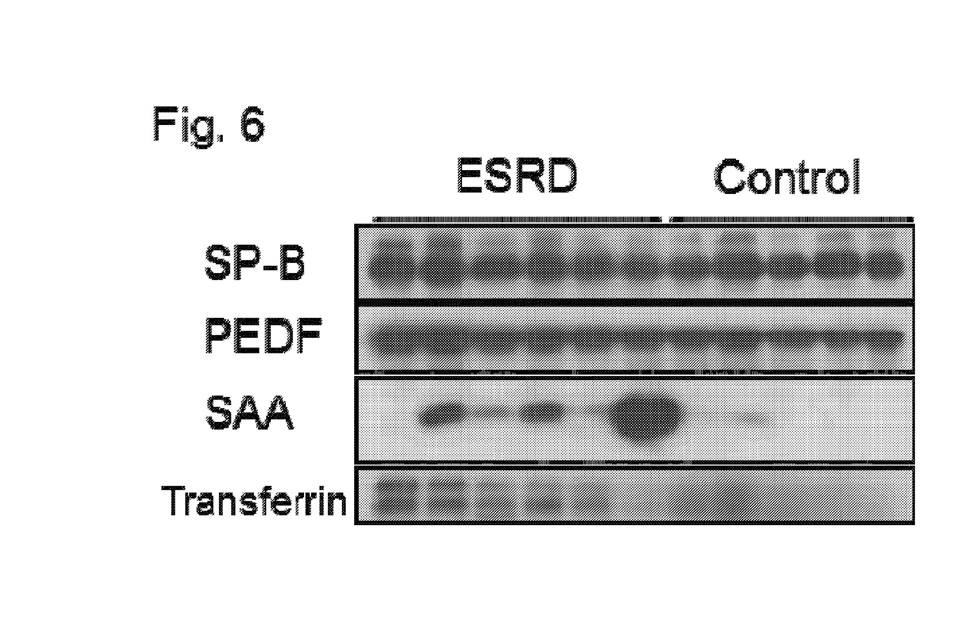

Fig.6 shows the immunodetection of MS-identified proteins in the plasma of both ESRD patients and healthy subjects. Shown are immunoblots of SP-B, PEDF, SAA, and serotransferrin in 10 µg whole plasma of 6 ESRD and 6 healthy controls.

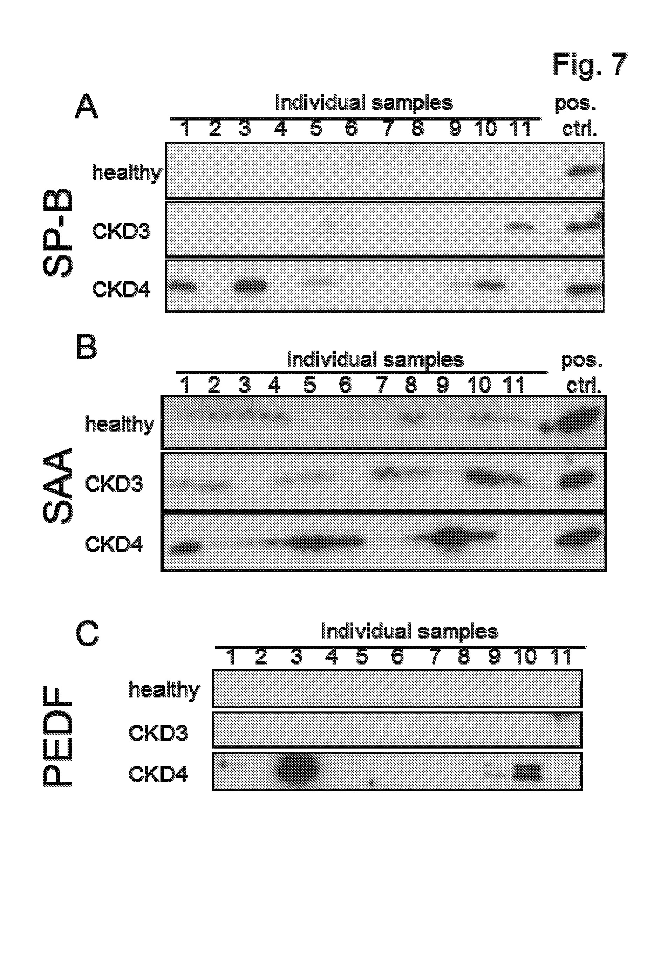

Fig.7 shows the immunodetection of HDL-associated proteins in healthy subjects and patients with CKD3 or CKD4. Immunoblot of (A) SP-B, (B) PEDF, and (C) SAA in 10 µg HDL samples.

### Examples:

### Materials and Methods:

### Subjects

The study was approved by the ethics committee of the General Hospital Vienna according to the declaration of Helsinki (EK 407/2007). Informed consent was obtained from each patient. A total of 10 stable patients with ESRD undergoing maintenance hemodialysis for a minimum of 3 months were recruited for the study. Patients were in a stable condition and free from intercurrent illness and infection for at least 3 months. As confirmed by clinical examination, patients were in a good state of health, notably without signs of malnutrition or wasting. All the patients were dialyzed on standard bicarbonate basis (Fresenius, Germany) for 4-5 h three times weekly using biocompatible polysulfone hemodialysis membranes. Dialysis adequacy was estimated using Kt/V values > 1.2 in all patients. None of the patients had residual renal function (diuresis over 24 h below 100 ml). The venous blood sample was drawn at midweek before dialysis session. Blood was immediately placed on ice and centrifuged at 4,000xg for 5 min at 4°C to obtain plasma. A group of 10 subjects with normal kidney function were used as controls. The clinical and biochemical characteristics of the patients and control subjects are given in Table 1.

**Table 1. Clinical characteristics of the study subjects used in the proteomics study.**

| | proteomics cohort | | replica cohort | |
|---|---|---|---|---|
| | ESRD | Control | ESRD | Control |
| n | 10 | 10 | 14 | 12 |
| Age (years) | 62.5(12.4) | 55.5(11.5) | 56.1 (18.8) | 37.0(11.5) |
| Weight (kg) | 77.8(15.5) | 84.4(11.2) | | |
| Gender (male/female) | 6/4 | 5/5 | 7/7 | 10/2 |
| Cholesterol (mg/dl) | 198.4 (59.5) | 138.5 (30.4) | 165.8 (40.6) | 198.8 (27.2) |
| Triglycerides (mg/dl) | 178.8 (45.3) | 98.8 (15.8) | 145.4 (64.4) | 92.8 (54.6) |
| HDL-cholesterol (mg/dl) | 36.5 (12.8) | 45.1(6.7) | 47.6 (12.6) | 64.5 (14.0) |
| LDL-cholesterol (mg/dl) | 143.3 (38.3) | 88.5(28.6) | 94.9 (37.8) | 115.8 (23.0) |
| Albumin (g/l) | 34(7.1) | 49.5(5.1) | 36.4 (4.5) | 47.1(2.8) |
| Blood urea nitrogen (mg/dl) | 128.7 (39.2) | 40.4 (7.9) | 57.4 (18.6) | 13.6 (2.3) |
| Creatinine (mg/dl) | 7.8(2.9) | 0.89 (0.2) | 8.4(2.4) | 1.0(0.1) |

### Isolation of High-Density Lipoproteins

HDL was isolated from fresh human plasma by sequential ultracentrifugation at a density d of 1.125<d<1.210 kg/L, respectively (Havel et al., J Clin Invest 34 (1955): 1345-53). In brief, the density of plasma was raised by addition of KBr (Merck, Darmstadt, Germany) to 1.125 kg/L, and ultracentrifugation was carried out for 12 h at 20°C in a 50.4 Ti rotor at 50,000 rpm using an Optima L-90-K ultracentrifuge (Beckman, Fullerton, CA). After removing the supernatant with the larger lipoproteins, the density of the infranate fraction was raised to 1.210 kg/L by further addition of KBr. HDL was then collected from the top of each polycarbonate thick wall centrifuge tube (Beckman) after another centrifugation step in the 50.4 Ti rotor at 50,000 rpm for 12 h.

### 1D-PAGE for subsequent shotgun analysis

The different protein fractions were loaded on 12% polyacrylamide gels, electrophoresis was performed until complete separation of a pre-stained molecular marker (Dual Color, Biorad, Hercules, CA) was visible. Gels were fixed with 50% methanol/10% acetic acid and subsequently silver stained as described below. Gel lanes were cut out of the gel into 6 bands and digested with trypsin as described below.

### Tryptic digest

Gel-pieces were destained with 15 mM K₃Fe (CN) ₆/50 mM Na₂S₂O₃ and intensively washed with 50% methanol/10% acetic acid. The pH was adjusted with 50 mM NH₄HCO₃, and proteins were reduced with 10 mM DTT/50 mM NH₄HCO₃ for 30 min at 56°C and alkylated with 50 mM iodacetamide/50 mM NH₄HCO₃ 20 min in the dark. Afterwards the gel-pieces were treated with acetonitril and dried in a vacuum centrifuge. Between each step, the tubes were shaken 5-10 min (Eppendorf Thermomixer comfort). Dry gel-spots were treated with trypsin 0.1 mg/ml (Trypsin sequencing grade, Roche Diagnostics) /50 mM NH₄HCO₃, in a ratio of 1:8 for 20 min on ice, afterwards covered with 25 mM NH₄HCO₃ and were subsequently incubated overnight at 37°C. The digested peptides were eluted by adding 50 mM NH₄HCO₃, the supernatant was transferred into silicon-coated tubes, and this procedure was repeated two times with 5% formic acid/50% acetonitrile. Between each elution step the gel-spots were ultrasonicated for 10 min. Finally the peptide solution was concentrated in a vacuum centrifuge to an appropriate volume.

### Mass spectrometry analysis

Mass spectrometry was performed as described previously. ⁵⁷ Peptides were separated by nano-flow LC (1100 Series LC system, Agilent) using the HPLC-Chip technology (Agilent) equipped with a 40 nl Zorbax 300SB-C18 trapping column and a 75 µm x 150 mm Zorbax 300SB-C18 separation column at a flow rate of 400 nl/min, using a gradient from 0.2% formic acid and 3% ACN to 0.2% formic acid and 50% ACN over 60 min. Peptide identification was accomplished by MS/MS fragmentation analysis with an iontrap mass spectrometer (XCT-Ultra, Agilent) equipped with an orthogonal nanospray ion source. The MS/MS data were interpreted by the Spectrum Mill MS Proteomics Workbench software (Agilent; version A.03.03.081), including peak list-generation and search engine, allowing for two missed cleavages and searched against the SwissProt Database for human proteins (Version 14.3 containing 20,328 protein entries) allowing for precursor mass deviation of 1.5 Da, a product mass tolerance of 0.7 Da and a minimum matched peak intensity (%SPI) of 70%. Due to previous chemical modification, carbamidomethylation of cysteine was set as fixed modification, Methionine oxidation was allowed as variable modification.

Estimated error rates were calculated from reversed database searches as previously described (Wimmer et al., Electrophoresis 30 (2009): 2076-2089). From this a protein valid was automatically assigned if it was identified with at least one specific peptide scoring above 13.0, which does not occur in other proteins identified in same samples. Protein identifications not fulfilling that requirement were selected manually as previously described (Slany et al., J Proteome Res 9 (2010): 6-21) whereby selection of protein isoforms was essentially performed as described by Zhang et al. (J Proteome Res 5 (2006): 2909-2918). Only peptides scoring above 9.0 were entered into the database.

### Peptide index

For quantitative spectral counting (Zhang et al., J Proteome Res 5 (2006): 2909-2918) the number of specific spectral peptide counts assigned to one protein across all fractions of a sample was summed and normalized against the sum of all identified specific peptide counts in that sample. Normalized spectral counts were multiplied with the mean value of total specific spectral peptide counts per patient. Finally these normalized spectral counts were used to calculate the peptide index for each protein identified by MS/MS, as follows: [(peptides in ESRD subjects/total peptides) × (% of ESRD subjects with ≥1 peptide)] - [(peptides in control subjects/total peptides) × (% of control subjects with ≥1 peptide)]. A peptide index of less than -0.4 or greater than 0.4, respectively, was chosen to identify selective enrichment of proteins in control subjects or ESRD subjects as described previously (Vaisar et al., J Clin Invest 117 (2007): 746-756).

### Immunoblotting

Reduced HDL or plasma preparations (10 µg per lane) separated by 10% SDS-PAGE were transferred to a nitrocellulose membrane and probed overnight at 4° C with primary antibodies as follows: SP-B and SAA (Santa Cruz Biotechnology), apoC-II (Gen-Script), apoA-I (Cell signaling), PEDF (R&D Biosystems) and serotransferrin (Biodesign International). Blots were washed, incubated with secondary horseradish peroxidase-conjugated antibodies as appropriate and developed using ECL Western blotting detection reagents (Amersham).

### Oxidation of HDL and HPLC analysis

Oxidation of HDL and analysis of oxidized HDL by HPLC was essentially performed as described (Wang et al., J Lipid Res 50 (2009): 586-594; Garner et al., J Biol Chem 273 (1998): 6080-6087). Briefly, fresh human blood was collected into heparinized vacutainers directly on ice, and plasma was immediately obtained by centrifugation at 1,000 g for 15 min at 4°C and stored until use at -80° C. HDL was isolated from the plasma by sequential ultracentrifugation. Native HDL (1-1.5 mg protein/ml) was oxidized in 20 mM PBS containing 100 µM diethylene triamine pentaacetic acid under air and at 37°C by exposure to the peroxyl radical generator AAPH (2 mM) for 2 h. The reaction was terminated by addition of butylated hydroxytoluene (100 µM). The reaction mixture (1 ml) was then passed through a gel filtration column (3 ml, NAP-10, GE Healthcare) eluted with 1.5 ml of PBS. The oxidized HDL was analyzed within 24 h. For HPLC analysis, freshly isolated HDL (0.06 mg protein) or differently oxidized HDL (1 mg protein) was subjected to a C18 column (250 × 4.6 mm, 5 µm, Vydac) with guard (5 µm, 4.6 ID, Vydac) eluted at 50°C and 0.5 ml/min, with the eluant monitored at 214 nm as described (Wang et al., J Lipid Res 50 (2009): 586-594).

### Statistics

For proteins that appeared enriched by the peptide index, Student's unpaired 2-tailed t test was used to compare the number of unique peptides identified in ESRD patients versus control subjects. For proteins found in only 1 group of subjects, a 1-sample t test was used to compare the number of unique peptides to a theoretical mean of 0. HPLC analysis of (oxidized) apoA-I/II is expressed as means ± SEM and was compared using Student's t test. For all statistical analyses, P < 0.05 was considered significant.

### Results

### Lipoprotein profile of ESRD patients

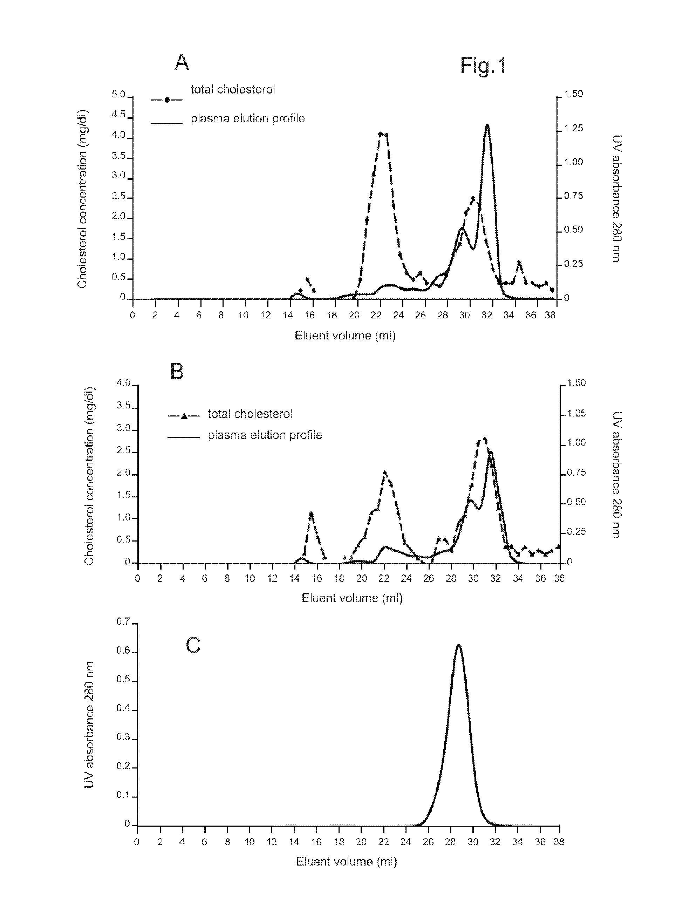

It was speculated that uremic HDL may possess an altered oxidation status or protein cargo. First, the lipoprotein profile of healthy controls and ESRD patients was defined by fast protein liquid chromatography (FPLC) analysis of whole plasma (Figure 1, A and B). The corresponding elution profile displayed 4 main peaks representing the major lipoprotein fractions very low-density lipoprotein (VLDL), LDL, HDL₂, HDL₃, whereas total cholesterol levels in the plasma fractions displayed three main peaks representing VLDL, LDL and HDL₂/HDL₃, respectively (Figure 1, A and B). As expected, the profile of ESRD patients showed reduced levels of HDL₂ and HDL₃ (Figure 1, A and B). Next HDL was isolated by conventional sequential ultracentrifugation (UC; Havel et al., 1955). The purified HDL eluted in a single peak demonstrating that UC allows high purification of HDL (Figure 1C).

### Assessment of the oxidation status of HDL in ESRD patients

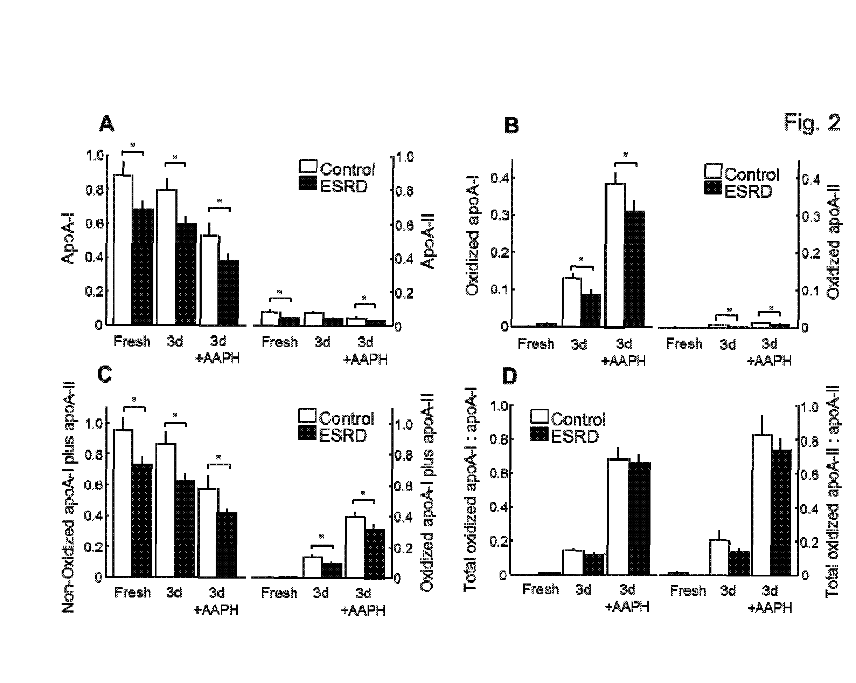

Oxidized HDL has been implicated in promoting atherosclerosis and oxidative stress contributes to morbidity and mortality in ESRD patients. Moreover, it has been suggested that the presence of oxidized HDL in ESRD patients may be causally linked to increased atherogenesis and therefore cardiovascular complications of uremia. As this assumption has not been vigorously tested, it was analyzed if oxidized HDL is present in ESRD patients by assessing the levels of total and oxidized apoA-I as well as ApoA-II by high pressure liquid chromatography (HPLC). A decrease in the relative content of apoA-I and apoA-II was detected in freshly isolated HDL from 15 ESRD patients compared to 15 controls (Figure 2A). Surprisingly, it was not possible to detect any significant amount of oxidized apoA-I or apoA-II in freshly isolated HDL neither in ESRD patients nor in healthy controls (Figure 2B). To further determine if there might be a difference in the susceptibility to oxidation of HDL between the two groups, *in vitro* oxidation with 2,2'-azobis-2-methylpropanimidamide (AAPH), a peroxyl radical generator, was performed with isolated HDL after three days of storage at 4°C. Interestingly, storing HDL samples alone significantly increased the content of oxidized apoA-I/II in HDL samples, irrespective of whether they were derived from control or ESRD patients (Figure 2B). Oxidation with AAPH for 2h further augmented the extent of apoA-I/II oxidation (Figure 2B). Oxidation was associated with the expected loss of non-oxidized apoA-I/II (Figure 2C). Compared with HDL from controls, HDL from ESRD patients accumulated less oxidized apoA-I and apoA-II. However, when expressed as relative to the total amount of apoA-I/II, there was no difference between HDL from controls and ESRD patients (Figure 2D). These results show that HDL in plasma from ESRD patients is not significantly oxidized *in vivo* and, moreover, it is not more vulnerable to oxidation than HDL from healthy controls.

### Identification of HDL-associated proteins by shotgun proteomics

As the oxidation status was similar between ESRD patients and healthy controls, it was investigated whether the protein cargo of HDL might reflect the qualitative impairment of HDL in uremia. To identify HDL-associated proteins, mass spectroscopy (MS) of purified HDL from 10 patients on maintenance hemodialysis and from 10 healthy controls (Table 1) was performed. As depicted in table 1, HDL-C concentrations were lower, while triglycerides, total cholesterol, and LDL-cholesterol (LDL-C) were elevated in ESRD patients. The HDL samples from the ESRD patients and the control subjects were further analyzed by one dimensional gel electrophoresis followed by electrospray ionization (ESI)-Ion Trap MS. The following criteria were used to identify proteins: a high peptide identification score (see Methods) and at least 3 peptides corresponding to a protein of interest had to be detected in at least 3 subjects. With these criteria 64 proteins could be identify that were HDL-associated (Table 2). 46 proteins were identified that have been previously described, whereas 18 novel proteins were discovered as HDL-proteins. This approach revealed all HDL apolipoproteins except apoA-V and apoC-IV and many proteins that have previously been identified in HDL indicating that the overall experimental approach according to the present invention was valid (Table 2). Importantly, the present findings were substantiated by identifying some of these proteins such as serotransferrin and SAA to be present in HDL from independent cohort samples by immunoblot analysis (Figure 3). The newly identified HDL-associated proteins included proteins such as alpha-1-acid glycoprotein 1 (AGP 1), zinc-alpha-2-glycoprotein (AZGP1), surfactant-associated protein B (SP-B), pigment epithelium-derived factor (PEDF), antithrombin-III or complement factor D (CFD) (Table 2). SP-B and PEDF were verified to be HDL-associated by immunoblotting HDL samples from an independent patient cohort (Figure 3).

**Table 2. Proteins detected by ESI-Ion trap MS in HDL from 10 ESRD patients and 10 healthy control subjects.**

| | | No of peptides | | Subjects with detectable proteins | | |
|---|---|---|---|---|---|---|
| AccNr | Name | ESRD | Control | ESRD | Control | References |
| Previously identified HDL-associated proteins | | | | | | |
| P02647 | ApoA-I | 416 | 375 | 10 | 10 | 1,2,3,5 |
| P02768 | Serum albumin | 252 | 222 | 10 | 9 | 1,2,5 |
| P06727 | ApoA-IV | 206 | 125 | 10 | 9 | 1,2,3,5 |
| P02649 | ApoE | 154 | 111 | 10 | 10 | 1,2,3,5 |
| P05090 | ApoD | 96 | 75 | 10 | 10 | 1,2,5 |
| P02652 | ApoA-II | 85 | 75 | 10 | 10 | 1,2,3,5 |
| P27169 | PON1 | 62 | 62 | 10 | 10 | 1,2,3,5 |
| P02656 | ApoC-III | 63 | 43 | 10 | 10 | 1,2,3,5 |
| P35542 | SAA4 | 54 | 49 | 10 | 10 | 2 |
| P02735 | SAA | 58 | 25 | 10 | 7 | 1,2,5 |
| P02654 | ApoC-I | 37 | 38 | 10 | 9 | 2,3 |
| 095445 | ApoM | 41 | 32 | 10 | 10 | 1,2,3,5 |
| O14791 | ApoL | 36 | 34 | 10 | 10 | 1,2,3,5 |
| P02787 | Serotransfernn | 35 | 23 | 6 | 5 | 1,2 |
| P00738 | Haptoglobin | 30 | 25 | 6 | 6 | 1,4 |
| P00739 | Haptoglobin-related protein (HRP) | 26 | 29 | 7 | 7 | 2,5 |
| P10909 | Clusterin | 26 | 22 | 9 | 7 | 2,3 |
| P02655 | ApoC-II | 37 | 10 | 10 | 5 | 1,2,5 |
| P02766 | Transthyretin (TTR) | 22 | 19 | 6 | 5 | 1,2,3,5 |
| P68871 | Hemoglobin (subunit beta) | 16 | 12 | 7 | 4 | 4 |
| Q15166 | PON3 | 14 | 14 | 8 | 7 | 2,5 |
| P01857 | Ig gamma-1 chain C region (IGHG1) | 15 | 13 | 5 | 5 | 3 |
| Q13790 | ApoF | 15 | 11 | 9 | 9 | 2,5 |
| P02760 | AMBP | 23 | 1 | 5 | 1 | 2 |
| P55058 | Phospholipid transfer protein (PLTP) | 13 | 10 | 8 | 6 | 2,5 |
| P04217 | Alpha-1B-glycoprotein (A1BG) | 12 | 10 | 5 | 5 | 1,2 |
| P02749 | Apo-H (Beta-2-glycoprotein 1) | 18 | 3 | 5 | 1 | 2 |
| P04180 | LCAT | 13 | 8 | 6 | 3 | 2 |
| P02774 | Vitamin D-binding protein (DBP) | 13 | 7 | 6 | 5 | 2,3 |
| P04114 | ApoB-100 | 7 | 13 | 5 | 3 | 2,3 |
| P02765 | Alpha-2-HS-glycoprotein (AHSG) | 8 | 9 | 6 | 5 | 2,3 |
| P04004 | Vitronectin (VTN) | 5 | 9 | 4 | 4 | 2,3 |
| P01024 | Complement C3 | 7 | 5 | 4 | 5 | 1,2,3 |
| P0C0L4 | Complement C4-A | 5 | 6 | 4 | 5 | 2 |
| P08519 | Apo(a) | 2 | 9 | 2 | 3 | 5,6 |
| P19652 | Alpha-1-acid glycoprotein 2 (AGP 2) | 6 | 4 | 4 | 3 | 2 |
| P36955 | Pigment epithelium-derived factor (PEDF) | 7 | 3 | 2 | 1 | 3 |
| P36955 | Serpin F1 | 7 | 3 | 2 | 1 | 2 |
| P69905 | Hemoglobin (subunit alpha) | 6 | 3 | 4 | 2 | 4 |
| Q96QR1 | Secretoglobin family 3A member 1 (Ugrp2) | 2 | 5 | 1 | 4 | 7 |
| Q9UHG3 | Prenylcysteine oxidase 1 (PCYOX1) | 3 | 3 | 3 | 3 | 2 |
| P02790 | Hemopexin | 3 | 3 | 2 | 3 | 2,3 |
| P00450 | Ceruloplasmin (CP) | 1 | 5 | 1 | 2 | 1 |
| P02775 | Platelet basic protein (PBP) | 1 | 5 | 1 | 3 | 5 |
| P01008 | Antithrombin-III | 0 | 4 | 0 | 3 | 3 |
| P22614 | SAA3P | 4 | 0 | 3 | 0 | 8 |
| Proteins identified in this study as HDL associated | | | | | | |
| P02763 | Alpha-1-acid glycoprotein 1 (AGP 1) | 15 | 12 | 6 | 5 | |
| P25311 | Zinc-alpha-2-glycoprotein (AZGP1) | 9 | 6 | 3 | 2 | |
| P07988 | Surfactant protein B (SP-B) | 11 | 0 | 7 | 0 | |
| P41240 | c-src tyrosine kinase CSK | 6 | 4 | 6 | 4 | |
| P63261 | Actin, cytoplasmic 2 | 1 | 9 | 1 | 2 | |
| P00746 | Complement factor D (CFD) | 7 | 1 | 3 | 1 | |
| P60985 | KRTDAP | 3 | 2 | 3 | 2 | |
| Q02818 | Nucleobndn-1 | 2 | 3 | 2 | 3 | |
| Q8IW75 | Serpin A12 | 3 | 0 | 3 | 0 | |
| Q9UKF2 | ADAM 30 | 3 | 0 | 3 | 0 | |
| Q9UP83 | COG complex subunit 5 (COG5) | 3 | 0 | 3 | 0 | |
| Q12907 | Vesicular integral-membrane protein VIP36 | 2 | 1 | 2 | 1 | |
| Q8ND99 | Protein C19orf16 | 2 | 1 | 2 | 1 | |
| Q8NHR9 | Profilin IV | 2 | 1 | 2 | 1 | |
| Q8TBF8 | Protein FAM81A | 2 | 1 | 2 | 1 | |
| Q9UHN6 | Transmembrane protein 2 | 1 | 2 | 2 | 1 | |
| P19320 | Vascular cell adhesion protein 1 (V-CAM1) | 1 | 2 | 1 | 2 | |
| Q13477 | MADCAM1 | 1 | 2 | 1 | 2 | |

References in table 2:
1: Rezaee et al., Proteomics, 6 (2006): 721-730.
2: Vaisar et al., J Clin Invest, 117 (2007): 746-756.
3: Gordon et al., J Proteome Res 9 (2010): 5239-5249.
4: Watanabe et al., J Biol Chem 284 (2009): 18292-18301.
5: Davidson et al., Arterioscler Thromb Vasc Biol 29 (2009): 870-876.
6: Vaisar et al., Clin Chim Acta, 411 (2010): 972-979.
7: Bin et al., J Immunol 171 (2003): 924-930.
8: Meek et al., Proc Natl Acad Sci U S A 89 (1992): 7949-7952.

### Gene ontology analysis identifies iron and heme proteins linked to HDL

Previously, HDL-associated proteins have been linked to lipid metabolism, acute-phase response and innate immunity. Therefore, gene ontology (GO) analysis was performed to obtain a deeper understanding if the identified proteins according to the present invention likewise link into these clusters and if additional functional categories can be recognized. As anticipated, many of the identified proteins (21 of 63) were linked to cholesterol and lipoprotein metabolism (Figure 4). Some proteins were identified as regulators of complement activation, the acute-phase response, and also linked to protein breakdown processes (Figure 4). Moreover, prenylcysteine oxidase (PCYOX1) and ceruloplasmin (CP) were found that are implicated in the regulation of LDL oxidation (Figure 4). Unexpectedly, 7 proteins were identified, which directly regulate heme/iron metabolism such as hemopexin, serotransferrin, hemoglobin, α-1-microglobulin/bikunin precursor (AMBP), haptoglobin and haptoglobin-regulated protein (HRP, Figure 4). Interestingly, although heme/iron metabolism has been recently linked to atherogenesis, a critical regulatory role of HDL for this process is unknown at present. Moreover, three identified proteins belong to the Golgi complex compartment. As cholesterol efflux from cells to apoA-I involves mobilization of cholesterol from the trans-Golgi network, Golgi proteins may well become loaded to HDL by this route.

### The proteomic fingerprint of HDL from ESRD patients

Then the proteomic composition of ESRD-HDL was compared with HDL from individuals with normal kidney function. To quantify differences between the two groups, the previously described "peptide index", an empiric test based on peptide abundance to measure the relative protein abundance in different groups of subjects, was used. As presented in Figure 5, 4 proteins were identified that were significantly enriched in HDL from ESRD patients according to the peptide index: SP-B, apoC-II, SAA, and AMBP. Indeed, SP-B was exclusively detected in HDL from ESRD patients but not in healthy individuals (Figure 5 and Table 2). These results were supported by immunoblotting the enriched proteins in HDL from an independent cohort of healthy individuals and ESRD patients (Figure 3). Moreover, it was observed that various proteins, which were enhanced in the ESRD group but did not reach statistical significance according to the peptide index, were still highly enriched in the independent patient cohort such as serotransferrin or PEDF (Figure 3). These results show that distinct HDL-associated proteins are strongly enriched or are exclusively present in ESRD patients. The selective enrichment of these molecules in HDL during uremia creates the opportunity to consider them as potential novel biomarkers in uremic patients.

### SP-B and PEDF are selectively enhanced in HDL from ESRD patients

Next it was investigated whether the presence of the proteins enriched in ESRD-HDL merely reflects their occurrence in the plasma or whether these proteins are exclusively incorporated into the HDL of ESRD patients. Therefore, immunoblotting of plasma of the HDL-associated proteins was performed. Interestingly, SP-B and PEDF were present in the plasma of control and ESRD subjects to a similar amount, whereas SAA and serotransferrin were already enriched in the plasma of ESRD patients (Figure 6). These results show that SP-B and PEDF present in ESRD-HDL are incorporated by a particular pathogenic mechanism and cannot be explained by the mere presence of these proteins in the plasma.

### SP-B and PEDF become gradually enriched in HDL from CKD3 to CKD4

To discriminate between ESRD patients on maintenance dialysis and earlier stages of renal insufficiency it was investigated if the identified HDL-enriched proteins from ESRD patients can be detected in CKD stage 3 and 4. Consequently, HDL was isolated from 11 CKD3 and 11 CKD4 patients and subjected them to immunoblotting. Strikingly, while in CKD3 patients HDL-associated SP-B was detectably only in one patient, SP-B was already present in 5 out of 11 HDL samples of CKD4 patients (Figure 7A) This shows that the present invention is also specifically suitable for monitoring the progress of the disease in a given patient or patient collective over time. This also enables improved and more patient-specific medication and therapies, e.g. application of new therapies e.g. in case of risk of cardiovascular complications, in order to change the survival/therapy success expectations of "normally treated" patients. In contrast, HDL-associated SAA was already present in healthy controls, but noticeably increased in CKD4 patients (Figure 7B). PEDF was not found in the HDL of CKD3 patients, whereas 4 CKD4 patients displayed PEDF-enriched HDL (Figure 7C). These results suggest that the incorporation of SP-B, SAA or PEDF into HDL precedes ESRD and can already be detected in many CKD4 patients. Hence, these proteins can be used as biomarkers for subsequent kidney failure, disease progression, and possibly other clinical endpoints such as cardiovascular morbidity and mortality.

### Discussion

The molecular causes for the excessive cardiovascular morbidity and mortality of uremic patients are still only incompletely understood. Recently, it has been documented that HDL from patients with ESRD is dysfunctional and unable to inhibit oxidation of LDL, a cardinal feature of the antiatherogenic property of HDL. The molecular basis for the dysfunctionality of ESRD-HDL is still unresolved. In a first step to identify possible features that might discern healthy from uremic HDL, the molecular composition of the HDL proteome was analyzed by shotgun proteomics, a comprehensive determination of all HDL-associated proteins in ESRD patients. The analysis according to the present invention identified many proteins that have been previously assigned to uremia and CKD such as hemoglobin, serotransferrin, LCAT, CP, antithrombin-III, or apoA-IV, which is also a novel independent predictor of CKD progression. Interestingly, LCAT has been demonstrated to be significantly reduced in ESRD patients, however, the data according to the present invention show that LCAT levels are specifically enhanced in ESRD-HDL. Moreover, these results revealed that a specific and robust set of proteins such as SP-B, apoC-II, SAA, AMBP, serotransferrin, and PEDF define the molecular organization of HDL of ESRD patients. It was further detected that these proteins are also enriched in HDL that was isolated from a second independent set of 14 ESRD patients compared to 12 healthy controls, which strongly corroborates the initial proteomic analyses. Interestingly, the proteins more frequently enriched in HDL from ESRD patients do not overlap with HDL from coronary artery disease (CAD) patients where apoC-IV, PON1, C3, apoA-IV, and apoE are significantly augmented. These results emphasize a concept that HDL is not uniformly modified by dyslipidaemia independent of the underlying disease but that each disease or a distinct group of diseases is associated with characteristic disease-specific HDL particles.

Noticeably, the present study identified SP-B as HDL-associated protein exclusively in ESRD patients but not in healthy subjects. SP-B was present in plasma to similar amounts in controls and ESRD patients demonstrating that SP-B is incorporated from plasma into HDL exclusively in ESRD patients but not control subjects. Previously, plasma SP-B was identified as a novel biomarker in chronic heart failure (De Pasquale et al., Circulation 110 (2004): 1091-1096). Complications such as pulmonary edema and pleural effusion are common in chronic heart failure as well as in ESRD patients and it may be hypothesized that fluid overload and fluid retention that are common in maintenance hemodialysis patients and that are highly associated with cardiovascular mortality in ESRD patients, lead to pulmonary damage resulting in the release of surfactant including SP-B into the plasma that may become incorporated into the HDL of these patients due to distinct physicochemical properties of SP-B including their lipophilicity. The present results show that HDL-bound SP-B is a potent biomarker for cardiovascular sequelae in ESRD patients. In that line, it was remarkable to observe that SP-B became gradually enriched in patients with CKD3 and 4. Hence, whereas in healthy individuals HDL-bound SP-B was not observe, SP-B was detected in some CKD3 and in even more in CKD4 patients (Figure 7). Consequently, HDL-bound SP-B is an early indicator for fluid overload and/or the progression of CKD. Further studies are on the way to assess the potential of SP-B to serve as a biomarker in this respect.

Non-diabetic CKD patients have lower levels of plasma apoC-II, however, in this study it was found that apoC-II is highly enriched in HDL of ESRD patients but not in HDL of healthy subjects. As the acquisition of apoC-II from HDL by chylomicrons is essential for lipid metabolism, a defective transition of apoC-II from HDL to chylomicrons might be one reason for the dyslipidemia, which is also characterized by a defective clearance of chylomicrons, in ESRD patients.

The GO analysis of HDL-associated proteins of the present invention showed that HDL is a major regulator of heme/iron metabolism (Figure 4). 7 proteins were identified, which directly control heme or iron homeostasis. It has been shown previously that hemoglobin and haptoglobin are present in HDL to regulate inflammation, however, a broader role for HDL to regulate heme metabolism is unappreciated. A critical role for iron to drive atherogenesis has long been suggested, however, on the contrary, anemia has similarly been recognized as risk factor for CVD. Interestingly, erythropoietin may modulate HDL-C levels. Hence, a complex regulatory network between atherosclerosis and iron metabolism may involve HDL as central molecule to control both processes.

Evidence for the presence of oxidized HDL *in vivo* is abundant in atherosclerosis. ApoA-I in HDL recovered from atherosclerotic lesions show significant oxidative modifications. Studies of animals and humans have indicated that oxidized HDL can also be detected in plasma during atherosclerosis. Oxidized HDL not only loses critical atheroprotective functions, but might even acquire pro-inflammatory and pro-thrombotic properties. Although the presence of oxidized HDL in ESRD patients is commonly accepted, only one recent study tested this hypothesis experimentally. Hence, Honda et al. proposed that oxidized HDL is present in hemodialysis patients and is associated with protein wasting by measuring oxidized apoA-I in serum via a self-established monoclonal antibody. However, as apoA-I is found in LDL as well as in free form in serum, this study did could not directly assess oxidized apoA-I within HDL. Of note, it is extremely critical to process HDL as quickly as possible as HDL at 4° C undergoes rapid self-oxidation, which may adulterate the measurement results (Figure 2B). The present invention, however, provides clear evidence that HDL in plasma is not oxidatively modified in ESRD *in vivo.* Hence, the loss of the anti-inflammatory and anti-atherogenic functions of HDL during ESRD is potentially due to other reasons e.g. the altered proteome of

### ESRD-HDL.

In conclusion, the proteome of HDL from ESRD patients was comprehensively identified and compared to the HDL-proteome of healthy subjects. Novel HDL-associated proteins and proteins that are more frequently incorporated into the HDL of ESRD patients are provided with the present invention, such as SP-B, apoC-II, SAA, or AMPB. These molecules are novel biomarkers to improve the ability to monitor therapeutic responses, predict meaningful future events including cardiovascular complications, and identify individuals at increased risk for the development of ESRD before its clinical onset. Finally, the present study provides a framework for evaluating novel proteins and pathways such as heme/iron metabolism for a direct involvement in the pathogenesis of ESRD to study the functional consequences of the complex alterations in HDL of ESRD patients.

## Claims

1. Method for indicating a renal disease in a patient comprising the following steps:
- providing a blood, serum or plasma sample from said patient,
- isolating high density lipoprotein (HDL) from said sample to obtain isolated HDL,
- determining the protein content of at least one protein of the following group of proteins in said isolated HDL: Surfactant protein B (SP-B), ApoC-II, Serum amyloid A, AMBP, ApoH, Serpin A12, Serotransferrin, Pigment epithelium-derived factor (PEDF), ADAM30 and Complement factor D, preferably at least one of SP-B, ApoC-II, Serum amyloid A and AMBP, especially SP-B, wherein a renal disease is indicated in said patient if the determined protein content of said at least one protein is elevated in said isolated HDL compared with a healthy subject.

2. Method according to claim 1, **characterised in that** said isolating of HDL from said sample is performed by precipitation, ultracentrifugation, fast protein liquid chromatography (FPLC) or immunoseparation.

3. Method according to claim 1 or 2, **characterised in that** said determining the protein content of at least one protein of said group of proteins is performed by an enzyme linked immunoassay (ELISA), electrophoresis, immunoblotting, mass spectroscopy, immunoprecipitation, multiplex bead-based assays, or combinations of said methods.

4. Method according to any one of claims 1 to 3, **characterised in that** said isolating of HDL from said sample is performed by precipitation, wherein said precipitation is performed by heparin/MnCl₂, dextran sulphate, sodium phosphotungstate-MgCl₂ or PEG.

5. Method according to any one of claims 1 to 4, **characterised in that** said renal disease is chronic kidney disease stage 3, chronic kidney disease stage 4 or end-stage renal disease (ESRD; chronic kidney disease stage 5).

6. Method according to any one of claims 1 to 5, **characterised in that** said determined protein content of said at least one protein is elevated in said isolated HDL compared with a healthy subject by a peptide index of at least 0.2, preferably at least 0.4, especially at least 0.6.

7. Method according to any one of claims 1 to 6, **characterised in that** said determined protein content of said at least one protein is elevated in said isolated HDL compared with a healthy subject by at least 30%, especially by at least 50%, especially by at least 100%.

8. Kit comprising
- a container for a blood, serum or plasma sample,
- means for isolation of HDL from a blood, serum or plasma sample,
- one or more detection reagent or determination means for determining the protein content of at least one protein of the following group of proteins in isolated HDL of a sample: Surfactant protein B (SP-B), ApoC-II, Serum amyloid A, AMBP, ApoH, Serpin A12, Serotransferrin, Pigment epithelium-derived factor (PEDF), ADAM30 and Complement factor D, preferably at least one of SP-B, ApoC-II, Serum amyloid A and AMBP, especially SP-B.

9. Kit according to claim 8, **characterised in that** it additionally comprises standardisation means for determining said protein content, preferably a healthy blood, plasma or serum sample and/or a renal disease blood, plasma or serum sample, or written information indicating the protein contents in a healthy and/or a renal disease sample.

10. Kit according to claim 8 or 9, **characterised in that** said determination means for determining the protein content of at least one protein of said group of proteins in isolated HDL of a sample is a substrate in association with a detection reagent, preferably a membrane, a filter, a chip, a bead, a gel, a well, a tube, a plate, a pin, a channel, a pre, a trench, a microparticle or a capillary.

11. Method for evaluating the renal status in a patient having or being at risk of having a renal disease comprising the following steps:
- providing a blood, serum or plasma sample from said patient,
- isolating high density lipoprotein (HDL) from said sample to obtain isolated HDL,
- determining the protein content of at least one protein of the following group of proteins in said isolated HDL: Surfactant protein B (SP-B), ApoC-II, Serum amyloid A, AMBP, ApoH, Serpin A12, Serotransferrin, Pigment epithelium-derived factor (PEDF), ADAM30 and Complement factor D, preferably at least one of SP-B, ApoC-II, Serum amyloid A and AMBP, especially SP-B, wherein the renal status is evaluated, preferably as being a disease status or a non-diseased status, by comparison of the protein content of said at least one protein determined with the protein content of said at least one protein in a subject with a known renal status.

12. Use of Surfactant protein B (SP-B), ApoC-II, Serum amyloid A, AMBP, ApoH, Serpin A12, Serotransferrin, Pigment epithelium-derived factor (PEDF), ADAM30 or Complement factor D, preferably at least one of SP-B, ApoC-II, Serum amyloid A or AMBP, especially SP-B, in the diagnosis of a renal disease in a blood, serum or plasma sample.

13. Method for diagnosing a renal disease in a patient comprising the following steps:
- providing a blood, serum or plasma sample from said patient,
- isolating high density lipoprotein (HDL) from said sample,
- analysing the protein composition in said isolated HDL to obtain a HDL protein profile of said sample,
- comparing the HDL protein profile of said sample with a HDL protein profile of a healthy person and with a HDL protein profile of a patient with confirmed renal disease, and
- diagnosing a renal disease if the comparison of the HDL protein profile of said sample corresponds to said HDL protein profile of a patient with confirmed renal disease.

14. Method according to claim 13, **characterised in that** obtaining the HDL protein profile includes determining the protein content of at least one protein of the group consisting of Surfactant protein B (SP-B), ApoC-II, Serum amyloid A, AMBP, ApoH, Serpin A12, Serotransferrin, Pigment epithelium-derived factor (PEDF), ADAM30 and Complement factor D, preferably at least one of SP-B, ApoC-II, Serum amyloid A and AMBP, especially SP-B.

15. Method for diagnosing a renal disease in a patient comprising the following steps:
- providing a blood, serum or plasma sample from said patient,
- isolating high density lipoprotein (HDL) from said sample,
- determining the presence or absence of SP-B in said isolated HDL,
- optionally analysing the protein content of SP-B in said isolated HDL to obtain a HDL protein profile of said sample,
- diagnosing a renal disease if the presence of SP-B in said isolated HDL is determined.
